# EUROPÄISCHE PATENTSCHRIFT

(11) **EP 2 990 794 B1**
(45) Veröffentlichungstag und Bekanntmachung des Hinweises auf die Patenterteilung: **19.02.2020**
(21) Anmeldenummer: 15182285.5
(22) Anmeldetag: 25.08.2015
(51) Int. Cl.: G01N 33/04, G01N 30/88

(54) **VERFAHREN ZUR UNTERSCHEIDUNG "BIOLOGISCH" UND "KONVENTIONELL" ERZEUGTER MOLKEREIPRODUKTE**
METHOD FOR DISCRIMINATING BETWEEN ORGANIC AND CONVENTIONAL DAIRY PRODUCTS
PROCEDE DE DISTINCTION ENTRE DES PRODUITS DE MATIERE LAITIERE BIOLOGIQUES OU CONVENTIONNELS

(30) Priorität: 25.08.2014 DE 102014216854
(43) Veröffentlichungstag der Anmeldung: 02.03.2016
(73) Patentinhaber: Technische Universität Dresden, 01069 Dresden (DE)
(72) Erfinder: Hofmann, Thomas, 98716 Geraberg (DE); Schwarzenbolz, Uwe, Dr., 01328 Dresden (DE); Henle, Thomas, Prof. Dr., 01309 Dresden (DE)
(74) Vertreter: Kailuweit & Uhlemann Patentanwälte Partnerschaft mbB

(56) Entgegenhaltungen:
- NAILA AHMED ET AL: "Assay of advanced glycation endproducts in selected beverages and food by liquid chromatography with tandem mass spectrometric detection", MOLECULAR NUTRITION & FOOD RESEARCH, Bd. 49, Nr. 7, 9. Juni 2005 (2005-06-09), Seiten 691-699, XP055239890, DE ISSN: 1613-4125, DOI: 10.1002/mnfr.200500008
- PAUL J. THORNALLEY ET AL: "Quantitative screening of advanced glycation endproducts in cellular and extracellular proteins by tandem mass spectrometry", BIOCHEMICAL JOURNAL, Bd. 375, Nr. 3, November 2003 (2003-11), Seiten 581-592, XP055099138, ISSN: 0264-6021, DOI: 10.1042/BJ20030763
- POULSEN MALENE W ET AL: "Advanced glycation endproducts in food and their effects on health", FOOD AND CHEMICAL TOXICOLOGY, Bd. 60, 16. Juli 2013 (2013-07-16), Seiten 10-37, XP028713435, ISSN: 0278-6915, DOI: 10.1016/J.FCT.2013.06.052
- B. DEINUM ET AL: "Effects of drying temperature on chemical composition and in vitro digestibility of forages", ANIMAL FEED SCIENCE AND TECHNOLOGY, Bd. 46, Nr. 1-2, März 1994 (1994-03), Seiten 75-86, XP055239798, AMSTERDAM, NL ISSN: 0377-8401, DOI: 10.1016/0377-8401(94)90066-3
- TREMBLAY G F ET AL: "Estimating Ruminal Protein Degradability of Roasted Soybeans Using Near Infrared Reflectance Spectroscopy<1,2>", JOURNAL OF DAIRY SCIENCE, AMERICAN DAIRY SCIENCE ASSOCIATION, US, Bd. 79, Nr. 2, Februar 1996 (1996-02), Seiten 276-282, XP027048025, ISSN: 0022-0302 [gefunden am 1996-02-01]

## Beschreibung

Die Erfindung betrifft Verfahren zum Nachweis einer artgerechten Tierfütterung und zur Unterscheidung gemäß Verordnung (EG) Nr. 834/2007 (Öko-Verordnung) und nicht gemäß Verordnung erzeugter Molkereiprodukte.

Als "Bio-Lebensmittel" deklarierte Produkte müssen gemäß Verordnung (EG) Nr. 834/2007 (Öko-Verordnung) aus der sogenannten "ökologischen Landwirtschaft" stammen (d.h. kein Pestizideinsatz, keine gentechnischen Veränderungen usw.). Für landwirtschaftliche Nutztiere ist dabei festgelegt, dass sie "artgerecht" gehalten und ernährt werden. Diese Aspekte machen "Biolebensmittel" für zahlreiche Verbraucher insbesondere in den wohlhabenden Industrienationen sehr attraktiv. Die Nachfrage steigt ständig. So hat sich beispielsweise die Anlieferungsmenge an "Biomilch" im Zeitraum von 2000 bis 2012 von ca. 262.000 t auf 671.000 t mehr als verdoppelt (Milchindustrieverband, 2014).

Eine erfolgreiche Vermarktung von "Bio-Lebensmittel" setzt deren verlässliche Identifizierung und Unterscheidung von nicht gemäß Verordnung (EG) Nr. 834/2007 erzeugten Produkten voraus. Zur Absicherung der Erzeuger, vor allem aber zum Schutz der Verbraucher, die bereit sind, für "Bio-Lebensmittel" signifikant höhere Preise im Einzelhandel zu zahlen, werden Verfahren benötigt, die eine Kontrolle der Deklaration "biologisch" erzeugter Lebensmittel ermöglichen.

Aktuelle Verfahren zur Unterscheidung von "biologisch" und nicht gemäß Verordnung (EG) Nr. 834/2007 erzeugter ("konventionell") Milch beruhen auf der gaschromatographischen Untersuchung des Fettsäuremusters (Quantifizierung des Gehaltes an α-Linolensäure) oder der Analyse der Isotopenverteilung im Milchfett (Ermittlung der δ¹³C-Werte) (vgl. Molkentin und Giesemann, 2007). Beide Verfahren gehen dabei davon aus, dass die Zusammensetzung des Milchfettes maßgeblich durch das Futter der Kühe bestimmt wird. Kühe in "ökologischer Landwirtschaft" müssen mit Futter aus einem "geschlossenen Stoffkreislauf" gefüttert werden. Kraftfutter (u.a. Mais und andere proteinhaltige Pflanzen) wird nicht (oder nur wenig) zur Fütterung verwendet. Gemäß oben genannter Verordnung (EG) Nr. 834/2007 darf der Anteil an Kraftfutter maximal 40 % betragen. Es ist davon auszugehen, dass der Anteil an Kraftfutter künftig weiter reduziert wird. In der Schweiz sind seit 2014 Regelungen für Biomilcherzeuger in Kraft, die maximal 10 % Kraftfutter zulassen (FIBL, 2014). Das Grundfutter der Kühe soll vor allem aus Weidefutter (Gras, Klee) sowie Heu und Grassilage bestehen. Diese "C3-Pflanzen" bedingen einerseits den etwas höheren Gehalt an α-Linolensäure (der Mindestgehalt im Fett von "Biomilch" soll 0,5 % betragen), sowie einen δ¹³C -Wert von maximal -26,5 Promille.

Die genannten Verfahren sind zwar grundlegend für eine orientierende Charakterisierung von "Bio-Milch" geeignet, haben aber in der praktischen Anwendung unter anderem folgende nicht unerhebliche Probleme (vgl. Molkentin, 2012):
Um eine hinreichend gesicherte Unterscheidung zu ermöglichen ist ein hoher methodischer Aufwand notwendig. Insbesondere die Isotopenanalytik kann nur von wenigen Analysenlaboratorien durchgeführt werden, wobei spezielle Massenspektrometer nötig sind. Weitere wesentliche Parameter die das Analysenergebnis beeinflussen sind mögliche Störanfälligkeit sowie die Abhängigkeit von "zertifizierten" Standardmaterialien. Zudem ist ein hoher Anspruch an individuelle Erfahrung des Analysenpersonals notwendig.

Zudem ist die Identifizierung von "Bio-Milch" nur innerhalb bestimmter Grenzen möglich. Aufgrund produktabhängiger und jahreszeitliche Schwankungen des Fettgehaltes und der Fettzusammensetzung können für beide Verfahren nur "Schwellenwertbereiche" der jeweiligen Analysenparameter festgelegt werden.

Des Weiteren besteht die Gefahr "falsch positiver Werte". Hohe Gehalte an α-Linolensäure können auch bei nicht gemäß der Verordnung (EG) Nr. 834/2007 erzeugten Milchproben nach intensiver Weidewirtschaft trotz Zufütterung erheblicher Mengen an Kraftfutter vorkommen.

Ahmed et al. offenbart eine Methode zur Bestimmung von AGEs in Cola, verschiedenen Milchproben, Nagetierfutter sowie Urin (Ahmed et al., 2005). Weiterhin beschreibt Ahmed et al. den Einfluss der thermischen Prozessierung von Rohmilch auf deren Gehalt an AGEs, insbesondere N-ε-Carboxymethyllysin (CML), N-ε-Carboxyethyllysin (CEL) und Methylglyoxal Hydroimidazolon 1 (MG-H1).

Thornally et al. beschreibt ein Verfahren zur Quantifizierung von AGEs in zellulären und extrazellulären Proteinen mittels Tandemmassenspektrometrie, unter anderem 3-Desoxyglucosulose Hydroimidazolon (3DG-H), N-ε-Carboxyethyllysin (CEL), Glyoxal Hydroimidazolon (G-H), Methylglyoxal Hydro-imidazolon (MG-H) (Thornally et al., 2003).

Poulsen et al. offenbart das Vorkommen von AGEs in Nahrungsmitteln und deren Einfluss auf die Gesundheit (Poulsen *et al*., 2013). Weiterhin beschreibt Poulsen et al. den Gehalt von AGEs in Milchprodukten im Vergleich zu Kaffee und Backwaren sowie die Methoden zur Bestimmung von N-ε-Carboxymethyllysin (CML), N-ε-Carboxyethyllysin (CEL), Methylglyoxal Hydroimidazolon 1 (MG-H1), Pyrralin, Pentosidin und Ornithinoimidazolinon in Milchprodukten (ELISA, HPLC, RP-HPLC, LC-MS/MS oder FAB-MS). Poulsen et al. offenbart die Möglichkeiten der Abschätzung des Gesamt-AGE-Gehalt von Nahrungsmitteln durch die Bestimmung von Furosin oder CML und deren Grenzen.

Vor diesem Hintergrund ist ein Analysenverfahren in hohem Maße wünschenswert, mit dem eine eindeutige Identifizierung "biologisch" erzeugter Milch möglich wird.

Es ist daher die Aufgabe der vorliegenden Erfindung ein Verfahren anzugeben, welches eine Bestimmung von gemäß Verordnung (EG) Nr. 834/2007 erzeugten Produkten gewährleistet und die Nachteile im Stand der Technik überwindet.

Die Aufgabe wird durch ein Verfahren gemäß Anspruch 1 gelöst. Vorteilhafte Ausgestaltungen sind in den abhängigen Ansprüchen angegeben.

Erfindungsgemäß umfasst das Verfahren zur Bestimmung von gemäß Verordnung (EG) Nr. 834/2007 erzeugten Produkten die Schritte:
- Analyse der zu bewertenden Molkereiprodukt-Probe,
- Identifizierung von zumindest einem Glykierungsprodukt in der zu bewertenden Molkereiprodukt-Probe,
- Quantifizierung des identifizierten Glykierungsprodukts mit zumindest einer Referenzsubstanz,
- Ermittlung signifikanter Abweichungen zwischen quantifiziertem Glykierungsprodukt und produktspezifischen Referenzwerten, wobei als signifikante Abweichung des Gehalts an Glykierungsprodukt in der Molkereiproduktprobe eine Abweichung um das aus der Standardabweichung gerechnete Vertrauensintervall des Referenzwertes anzusehen ist.

Überraschend konnte festgestellt werden, dass eine Unterscheidung von "biologisch" und nicht gemäß Verordnung (EG) Nr. 834/2007 erzeugter, "konventioneller" Milch und daraus hergestellter Molkereiprodukte durch den direkten Nachweis definierter Futterbestandteile in der für den Endverbrauch bestimmten Konsummilch möglich ist. Es handelt sich dabei um bestimmte Aminosäurederivate (nachfolgend als "Glykierungsprodukte" bezeichnet), die im Verlauf der sogenannten Maillard-Reaktion ("Glykierung") zwischen Proteinen und Kohlenhydraten gebildet werden. Die Maillard-Reaktion zwischen Aminosäuren und Kohlenhydraten läuft vor allem während der thermischen Prozessierung von Futtermitteln (Kraftfutter) ab. Des Weiteren ist eine Bildung von Glykierungsprodukten während der Fermentation im Magen von Wiederkäuern wahrscheinlich.

Die Aminosäurederivate werden im Verdauungstrakt der Kuh aus dem Proteinverband freigesetzt, resorbiert und in bestimmten Mengen über die Milchdrüse in die Milch abgegeben. Glykierungsprodukte sind in "artgerechtem" Futter von Wiederkäuern (Weidefutter, Raufutter) nicht bzw. nur in sehr geringen Mengen vorhanden, da diese Futtermittel proteinarm und reich an Rohfaser (v.a. Cellulose) sind und nicht hitzebehandelt werden. Im Vergleich hierzu besteht Kraftfutter in der Regel aus thermisch behandeltem, eiweißreichen und stärkehaltigen Rohmaterialien (z.B. Soja- und Maisschrot, Melasse), aus welchen während der Fermentation im Wiederkäuermagen größere Mengen an Glykierungsprodukten freigesetzt werden können. Zudem können die gegenüber artgerechtem Futter hohen Mengen an reduzierenden Kohlenhydraten und Aminosäuren zu signifikant erhöhter Bildung von Glykierungsprodukten im Verdauungstrakt (v.a. Pansen) von Wiederkäuern und entsprechendem Transfer in die Milch führen.

Über die Analyse dieser thermisch induzierten Futterbestandteile in der für den Endverbraucher bestimmten Milch kann damit ein völlig neuer und unmittelbar an (evolutions)biologischen Grundlagen orientierter Nachweis einer artgerechten Fütterung und damit die Unterscheidung von "Bio-Milch" und "konventioneller" Milch und den daraus hergestellten Molkereiprodukten erfolgen.

Durch Quantifizierung der einzelnen Glykierungsprodukte unter Verwendung der jeweiligen Referenzsubstanz als internen Standard kann eine signifikante Aussage zur Unterscheidung von "Bio-Milch" und "konventioneller" Milch und daraus hergestellter Molkereiprodukte erfolgen. Dies erfolgt durch Bestimmung der Abweichung zwischen quantifiziertem Glykierungsprodukt und produktspezifischem Referenzwert, wobei als signifikante Abweichung des Gehalts an Glykierungsprodukt in der Molkereiproduktprobe eine Abweichung um das aus der Standardabweichung gerechnete Vertrauensintervall des Referenzwertes (Dean, 1951) anzusehen ist.

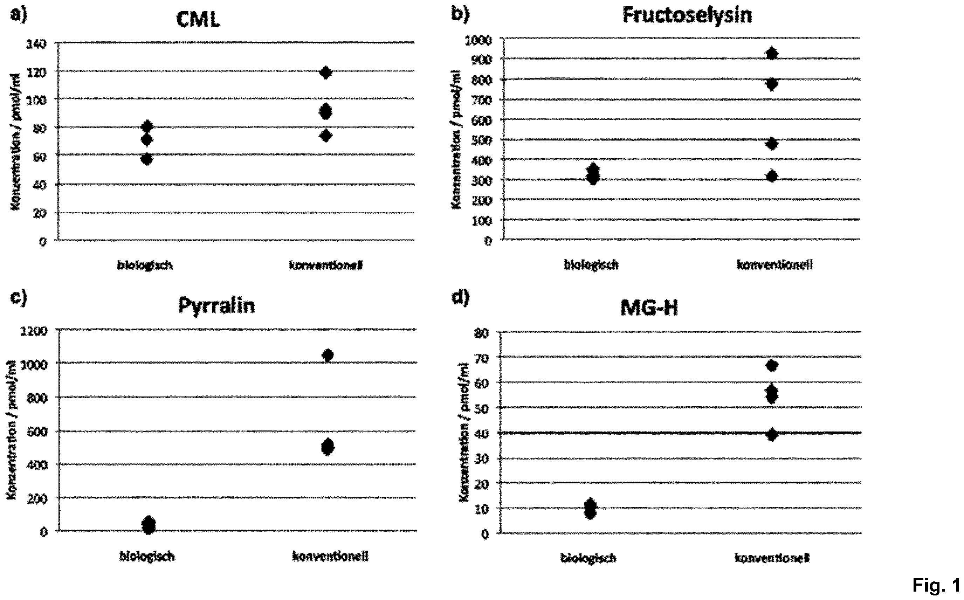

Als Indikatoren zur Unterscheidung von "Bio-Milch" und "konventioneller" Milch sind prinzipiell alle Aminosäurederivate der Maillard-Reaktion geeignet (Übersicht in Henle, 2005, Poulsen et al., 2013, vgl. Abb. 1).

Erfindungsgemäß ist das zu identifizierende Glykierungsprodukt Pyrralin (3) oder Methylglyoxal Hydroimidazolon (MG-H) (**4**). In einer Ausführungsform, in der zumindest zwei Glykierungsprodukte identifiziert werden, ist das zusätzlich zu identifizierende Glykierungsprodukt ausgewählt aus den nachfolgend wiedergegeben Verbindungen 1 bis 11: 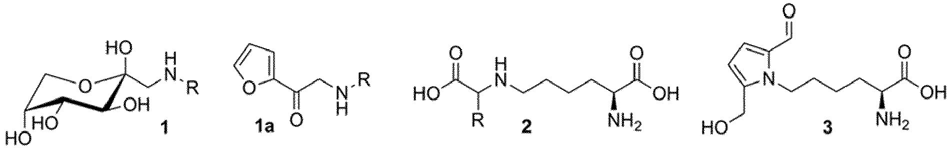 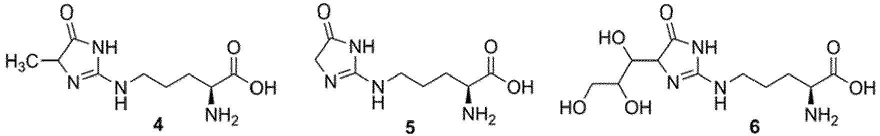 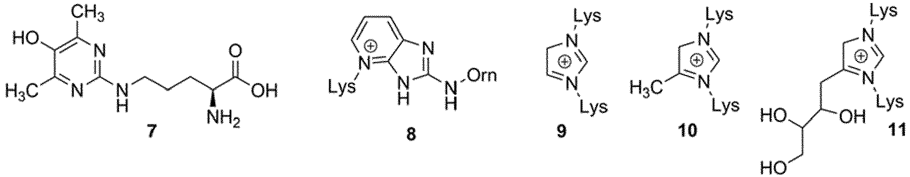 wobei die Verbindungen Amadori-Produkte von Aminosäuren (**1**), Furoylmethylaminosäuren (**1a**), N-ε-Carboxymethyllysin (CML) (**2**), Pyrralin (**3**), Methylglyoxal Hydroimidazolon (MG-H) (**4**), Glyoxal Hydroimidazolon (G-H) (**5**), 3-Desoxyglucosulose Hydroimidazolon (3DG-H) (**6**), Argpyrimidin (**8**), Glyoxal-Lysin Dimer (GOLD) (**9**), Methylglyoxal-Lysin Dimer (MOLD) (**10**) oder 3-Deoxyglucoson-Lysine Dimer (DOLD) (**11**); Lys = Lysinrest und Orn = Ornithinrest bezeichnen.

In einer Ausführungsform der Erfindung werden zumindest zwei Glykierungsprodukte in der zu bewertenden Molkereiproduktprobe identifiziert. Erfindungsgemäß ist dabei zumindest eines der zwei zu identifizierenden Glykierungsprodukte Pyrralin (**3**) oder Methylglyoxal Hydroimidazolon (MG-H) (**4**). Besonders bevorzugt werden zumindest die Glykierungsprodukte Pyrralin (3) und Methylglyoxal Hydroimidazolon (MG-H) (**4**) identifiziert.

In einer weiteren Ausführungsform der Erfindung erfolgt die Identifizierung des zumindest einen Glykierungsprodukts mittels Massenspektrometrie. Geeignete Massenspektrometrieverfahren umfassen dabei LC-MS, GC-MS oder MS/MS-Verfahren. Beispielsweise erfolgt die Identifizierung des zumindest einen Glykierungsprodukts mittels RP-HPLC und Massenspektrometrie.

Die vorbenannten erfindungsgemäßen Ausführungsformen sind geeignet die Aufgabe zu lösen. Dabei sind auch Kombinationen der offenbarten Ausführungsformen zur Lösung der Aufgabe geeignet. Bevorzugte Weiterbildungen der Erfindung ergeben sich aus den Kombinationen der Ansprüche.

Nachfolgend soll die Erfindung anhand einiger Ausführungsbeispiele und der zugehörigen Figur eingehender erläutert werden. Die Ausführungsbeispiele sollen die Erfindung beschreiben ohne sich auf diese zu beschränken.

Es zeigen die
Fig.1 eine grafische Darstellung der Ergebnisse der Quantifizierung der Verbindungen 1 bis 4 in "konventioneller" und "biologisch" erzeugter Milch, die
Fig. 2 ein Diagramm der bestimmten Konzentration der Verbindung 1 in Vergleichsproben, in
Fig. 3 ein Diagramm der bestimmten Konzentration der Verbindung 2 in Vergleichsproben, in
Fig. 4 ein Diagramm der bestimmten Konzentration der Verbindung 3 in Vergleichsproben und in
Fig. 5 ein Diagramm der bestimmten Konzentration der Verbindung 4 in Vergleichsproben.

In einem ersten Ausführungsbeispiel erfolgt die Probenvorbereitung zur Durchführung der Identifizierung der erfindungsgemäßen Glykierungsprodukte. Die quantitative Bestimmung freier (d.h. nicht proteingebundener) Glykierungsprodukte in Milch erfolgt mittels RP-HPLC und Massenspektrometrie nach Abtrennung der Proteinfraktion durch Fällung mit organischen Lösungsmitteln, Entfettung und anschließender Festphasenextraktion der Analyten.

Zur Aufarbeitung der Proben werden folgende Chemikalien bzw. Lösungen benötigt:
ACN/MeOH, 50:50, v/v, eisgekühlt (-25 °C), jeweils LC-MS-grade
- MeOH, LC-MS-grade
- Heptan, 99 %
- bidestilliertes Wasser
- Nonafluorpentansäure (NFPA), 97 %
- 10 mM NFPA (ca. 1,385 g NFPA add. 500 ml bidest. Wasser)
- MeOH/10 mM NFPA, 50:50, v/v
- MeOH/10 mM NFPA, 5:95, v/v
- MeOH/10 mM NFPA, 90:10, v/v

Zur Identifizierung und Quantifizierung der Glykierungsprodukte in den Proben wurde ein Mix isotopenmarkierter Standards verwendet. Diese wurden separat synthetisiert. Der beispielshaft nachfolgend dargestellte Standardmix enthielt die folgenden Referenzsubstanzen:

| **Standard** | **Konzentration / nmol/ml** |
|---|---|
| CML | 2,91 |
| MG-H1 | 1,65 |
| Pyrralin | 1,83 |
| FructoselysinL | 1,31 |

Zur Durchführung der Identifizierung und Aufarbeitung der Proben wurden für eine Doppelbestimmung jeweils 1000 µl Milch in ein PP-Röhrchen (Greiner Bio-One GmbH, 15 ml, mit Stopfen) pipettiert. Anschließend wurden 10 ml der eisgekühlten ACN/MeOH-Mischung (50:50, v/v, -25 °C) zugegeben. Danach erfolgte die Zugabe von 10 µl des oben beschriebenen Standardmixes. Das PP-Röhrchen wurde anschließend mit dem Stopfen verschlossen geschüttelt. Anschließend wurde die Mischung für 1 h bei -25 °C gekühlt. Danach wurden die Proben 5 min bei 3000 x g zentrifugiert (Heraeus Biofuge stratos auf 4 °C gekühlt). Der Überstand wurde in PP-Röhrchen dekantiert und über Nacht bei 20 °C im Stickstoffstrom getrocknet.

Anschließend wurden die Proben in 1,4 ml bidest. Wasser aufgenommen und 1,4 ml Heptan zugegeben. Nach Schütteln der Mischung erfolgte eine Zentrifugation für 2 min bei 1000 x g. Nach erfolgter Trennung der Phasen wurde die organische Phase abgenommen.

Anschließend erfolgte eine Festphasenextraktion mit Waters Oasis HLB 6 cc SPE Kartuschen. Die Säulen wurden zunächst durch Zugabe von 2 ml MeOH, 2 ml MeOH/10 mM NFPA (50:50, v/v) und 4 ml 10 mM NFPA konditioniert. Danach wurden 100 µl NFPA zur Probe zugegeben und nachfolgend gemischt. Die so vorbereitere Probe wurde auf die konditionierte Säule aufgebracht und mit 4 ml MeOH/10 mM NFPA (5:95, v/v) gewaschen. Anschließend erfolgte die Elution mit 5 ml MeOH/10 mM NFPA (90:10, v/v). Die eluierten Proben wurden über Nacht bei 20 °C im Stickstoffstrom getrocknet. Der verbleibende Rückstand wurde in 40 µl bidest. Wasser aufgenommen. Die so erhaltenen Proben wurden anschließend analysiert.

In einem weiteren Ausführungsbeispiel erfolgte die Analyse der vorbereiteten Proben mittels einer Agilent Series 1200 HPLC gekoppelt an ein Agilent 6410 Triplequadrupol-Massenspektrometer. Nachfolgend sind in Tab.1 die chromatographischen Bedingungen und in Tab. 2 die Parameter der Massenspektrometrie wiedergeben:

**Tab.1: Bedingungen RP-HPLC**

| **Parameter** | **Wert** |
|---|---|
| Säule | Kombination aus Agilent Zorbax C18 Rapid Resolution HT 2,1 x 50 mm, 1,8 µm und Phenomenex Hypercarb 2,1 x 150 mm, 5 µm |
| Injektionsvolumen | 10 µl |
| Temperatur | 35 °C |
| Flussrate | 0,2 ml/min |
| Fließmittel A | 5 mM NFPA in H₂O |
| Fließmittel B | 5 mM NFPA in Acetonitril |
| Gradient | 0,0 min; 15 % FM B |
| | 21,0 min; 85 % FM B |
| | 30,0 min; 85 % FM B |
| | 31,0 min; 15% FM B |
| | 55,0 min; 15% FM B |

**Tab. 2: Parameter der Massenspektrometrie**

| **Substanz** | **Vorläufer-Ion / m/z** | **Fragment-Ion / m/z** | **Art** | **Dwell-Time / ms** | **Fragm.Spannung /V** | **Kollisions-Energie / eV** |
|---|---|---|---|---|---|---|
| **CML** | 205 | 84 | Quantifier | 70 | 105 | 19 |
| **CML** | 205 | 130 | Qualifier | 70 | 105 | 7 |
| **13C-CML** | 207 | 84 | Quantifier | 100 | 105 | 19 |
| **13C-CML** | 207 | 130 | Qualifier | 100 | 105 | 7 |
| **MG-H** | 229 | 114 | Quantifier | 200 | 115 | 11 |
| **MG-H** | 229 | 166 | Qualifier | 200 | 115 | 11 |
| **13C-MG-H** | 235 | 121 | Quantifier | 200 | 115 | 11 |
| **13C-MG-H** | 235 | 171 | Qualifier | 200 | 115 | 11 |
| **Pyrralin** | 255 | 175 | Quantifier | 100 | 95 | 5 |
| **Pyrralin** | 255 | 148 | Qualifier | 100 | 95 | 5 |
| **13C-Pyrralin** | 263 | 182 | Quantifier | 100 | 95 | 5 |
| **13C-Pyrralin** | 263 | 153 | Qualifier | 100 | 95 | 5 |
| **FL** | 309 | 225 | Quantifier | 180 | 115 | 13 |
| **FL** | 309 | 273 | Qualifier | 180 | 115 | 11 |
| **FL** | 317 | 233 | Quantifier | 200 | 115 | 13 |
| **FL** | 317 | 281 | Qualifier | 200 | 115 | 11 |

Nachfolgend sind in Tab. 3 die Validierungsdaten der LC-MS-Methode für beispielhaft ausgewählte erfindungsgemäße Verbindungen wiedergegeben. Die Nachweis- und Bestimmungsgrenzen erlauben eine zuverlässige Quantifizierung mit guter Reproduzierbarkeit:

**Tab. 3: Validierungsdaten der LC-MS-Methode**

| Grenzen in pmol/ml | CML | Fructoselysin | MG-H1 | Pyrralin |
|---|---|---|---|---|
| NWG | 8,16 | 5 | 2,87 | 0,88 |
| EG | 16,32 | 10 | 5,74 | 1,76 |
| BG | 24,48 | 15 | 8,61 | 2,64 |
| CV | 0,85% | 4,27% | 0,92% | 0,44% |

| | | | | |
|---|---|---|---|---|
| NWG ... Nachweisgrenze, EG ... Erfassungsgrenze, BG ... Bestimmungsgrenze, CV ... Variationskoeffizient unter Wiederholbedingungen | | | | |

In einem weiteren Ausführungsbeispiel sind in der Fig. 1 beispielhaft die Ergebnisse der Analytik für die Verbindungen **1** bis **4** dargestellt. Die quantitativen Untersuchungen der Verbindungen **1** (R = N-ε-Lysyl) bis **4** ergaben für kommerziell erhältliche Konsummilchproben die in Fig. 1 dargestellten Ergebnisse. Die Gehalte der Glykierungsprodukte Fructoselysin (**1**) und Carboxymethyllysin (CML) (**2**) unterscheiden sich nicht signifikant zwischen "konventionellen" und "biologisch" erzeugten Milchproben (Fig. 1 a und b). Für Pyrralin (**3**) und MG-H1 (**4**) (Fig. 1 c und d) konnten jedoch signifikante Unterschiede erkannt werden: Beide Verbindungen waren in "biologisch" erzeugten Milchproben in nur sehr geringen Konzentrationen enthalten (Gehalte von MG-H1 (**4**) bis 3 µg/l, Gehalte von Pyrralin (**3**) bis 14 µg/l). In "konventionell" erzeugten Produkten lagen die Gehalte von MG-H1 (**4**) zwischen 9 und 15 µg/l und von Pyrralin (**3**) zwischen 125 und 270 µg/l.

Unterschiedliche Gehalte der einzelnen Glykierungsprodukte können auf deren unterschiedliche Bioverfügbarkeit zurückgeführt werden. Grundlagenuntersuchungen im Arbeitskreis (vgl. z.B. Förster und Henle, 2003; Förster et al., 2005; Hellwig et al., 2009; Hellwig et al., 2011; Hellwig et al., 2014) konnten zeigen, dass Fructoselysin und CML im Verdauungstrakt aus dem Nahrungsprotein freigesetzt, jedoch nicht resorbiert werden und folglich nicht in den Körper und damit die Milch gelangen können. Die in der Milch messbaren Gehalte (Abb. 1 a und b) sind vermutlich endogenen Ursprungs (d.h. aus dem "Turnover" glykierter Körperproteine stammend). Im Gegensatz hierzu konnte für Pyrralin (**3**) und MG-H1 (**4**) sowohl in Interventionsstudien als auch auf zellulärer Ebene nachgewiesen werden, dass beide Verbindungen aus alimentären Quellen zu hohen Anteilen resorbiert werden, was deren Transfer in die Milch erklärt (Förster und Henle, 2003; Förster et al., 2005).

Pyrralin (**3**) und MG-H1 (**4**) sind damit sehr gut geeignete Markersubstanzen für erhitzte Futtermittel. Die quantitative Bestimmung beider Verbindungen in der Milch erlaubt damit direkte Rückschlüsse auf die Verwendung thermisch prozessierter Futtermittel. Bei artgerechter Fütterung werden beide Verbindungen nur in sehr geringen Konzentrationen in der Milch nachweisebar sein, was als unmittelbarer Indikator für die "biologische" Erzeugung von Milch genutzt werden kann.

In einem weiteren Ausführungsbeispiel wurden Vergleichsuntersuchungen zwischen "konventionell" und "biologisch" erzeugten Milchproben untersucht. Dabei wurden die Gehalte der Glykierungsprodukte Fructoselysin (**1**), Carboxymethyllysin (CML) (**2**), Pyrralin (**3**) und MG-H1 (**4**) in den jeweiligen Milchproben bestimmt. Die Ergebnisse sind nachfolgend in der Tab. 4 dargestellt:

**Tab. 4**

| Probe | FL (1) [pmol/ml] | CML (2) [pmol/ml] | MG-H (4) [pmol/ml] | PYR (3) [pmol/ml] |
|---|---|---|---|---|
| K past. con. 1 | 481 | 77 | 40±5 | 492±14 |
| K past. con. 2 | 933 | 96 | 57±6 | 1056±67 |
| K UHT milk con. 1 | 318 | 125 | 67±6 | 520±15 |
| K UHT milk con. 2 | 778 | 76 | 54±5 | 514±17 |
| K raw milk no. 953 | n.e. | n.e. | 51±5 | 563±15 |
| K raw milk no. 999 | n.e. | n.e. | 44±3 | 659±10 |
| K ESL con.1 | -- | 94 ± 12,4 | 54±1 | 1295 ± 89 |
| K ESL con.2 | -- | -- | 38±6 | 480 ± 5 |
| K ESL con.3 | -- | -- | -- | 671±52 |
| K ESL con.4 | -- | -- | -- | 981±95 |
| K ESL con.5 | -- | -- | -- | 527±17 |
| K ESL con.6 | -- | -- | 41 ± 3 | 617±39 |
| K ESL con.7 | -- | -- | -- | 668±4 |
| B raw milk org. 1 | 318 | 57 | 8 ± 2 | 14±1 |
| B past. org. 1 | 353 | 73 | 12 ± 3 | 37±3 |
| B past. org. 2 | 307 | 83 | 11±4 | 53±4 |
| B past. org. 3 | 112 | 58 | 8±5 | 63±3 |
| B past. org. 4 | 271 | 63 | 10±4 | 85±11 |
| B past. org. 5 | 599 | 47 | 14±5 | 26±6 |
| B past. org. 6 | 960 | 67 | 9±4 | 48±10 |
| B past. org. 7 | 852 | 74 | 9±2 | 79±13 |
| B past. org. 8 | 336 | 62 | 10±3 | 80±4 |
| B past. org. 9 | 461 | 75 | 13±6 | 94±9 |
| B past. org. 10 | -- | 81 | 9 | 41±2 |
| B ESL org.1 | 354 | 79 | 17±5 | 191±19 |
| B ESL org.2 | -- | -- | 19±2 | 69±4 |
| B ESL org.3 | -- | -- | 11±2 | 64±5 |
| B ESL org.4 | -- | -- | -- | 116 ± 2,6 |
| B UHT milk org. 1 | 352 | 90 | 25±4 | 107±12 |

Dabei sind Milchproben aus "biologischer" Erzeugung mit einem "B" am Anfang gekennzeichnet, während "konventionell" erzeugte Milchproben mit "K" gekennzeichnet sind. Alle Analysen beziehen sich dabei auf freie Maillard-Reaktionsprodukte. Die Abkürzungen für die jeweiligen bestimmten Maillard-Reaktionsprodukte sind nachfolgend wiedergegeben:
FL: Fructoselysin (**1**); CML: N^{ε}-Carboxymethyllysin (**2**); Pyr: Pyrralin (**3**); MG-H: Methylhydroimidazolinon (**4**).

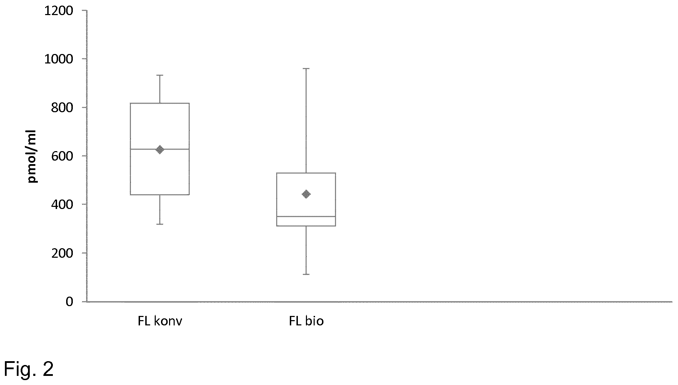

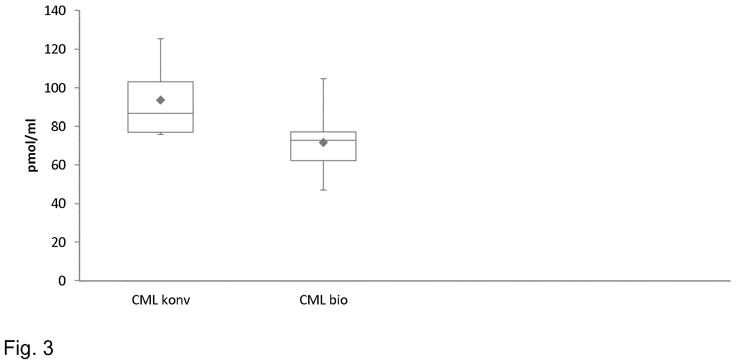

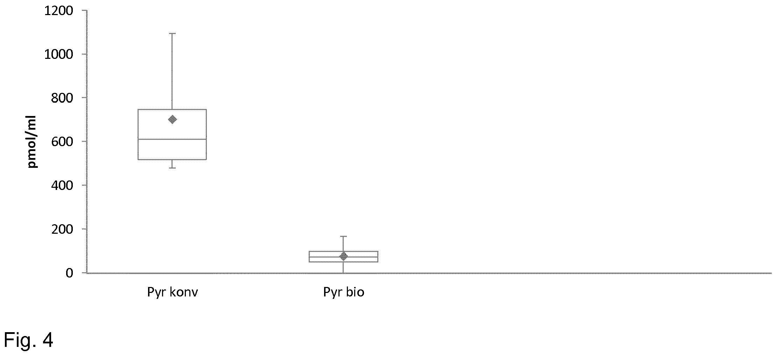

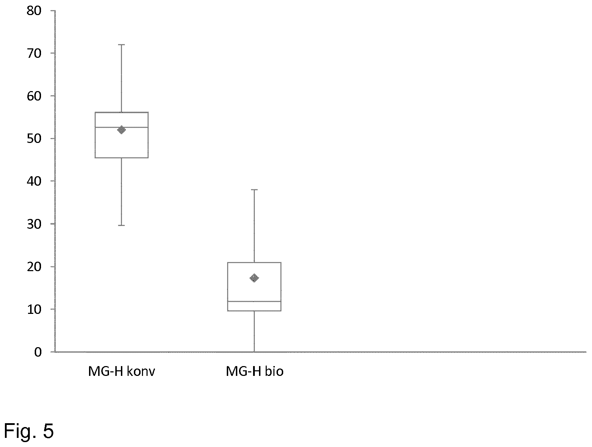

Die statistische Bewertung der Bestimmung der freien Maillard-Reaktionsprodukte in den Milchproben nach Tukey (1977) ist in den Fig. 2 - 5 dargestellt. Der Punkt in den jeweiligen Balken stellt dabei das arithmetische Mittel und die horizontale Linie den Median dar. Dabei ergibt sich insbesondere eine signifikante Abweichung der Gehalte von Pyrralin (3) in biologisch erzeugten und "konventionell" erzeugten Milchproben. Bei Methylhydroimidazolinon (4) zeigte sich eine weniger stark ausgeprägte Signifikanz während die Unterschiede bei Fructoselysin (1) und N^{ε}-Carboxymethyllysin (2) weniger stark ausgeprägt waren.

In einem weiteren Ausführungsbeispiel wurden Vergleichsuntersuchungen zwischen "konventionell" und "biologisch" erzeugten Joghurt untersucht. Die Ergebnisse dieser Vergleichsuntersuchungen sind in der Tab. 5 dargestellt.

| Probe | CML (2) [pmol/ml] | MG-H (4) [pmol/ml] | PYR (3) [pmol/ml] |
|---|---|---|---|
| BJ 1 | -- | 133 ± 5 | 102 ± 4 |
| BJ 2 | -- | 187 ± 18 | 58 ± 7 |
| BJ 3 | -- | 136 ± 3 | 119 ± 11 |
| BJ 4 | -- | -- | 103 ± 4 |
| BJ 5 | -- | -- | 73 ± 3 |
| BJ 6 | 875 ± 259,7 | -- | 216 ± 3 |
| KJ 1 | 94 ± 12,4 | 277 ± 12 | 1489 ± 4 |
| KJ 2 | -- | 152 ± 2 | 864 ± 49 |
| KJ 3 | -- | 250 ± 21 | 983 ± 39 |
| KJ 4 | -- | | 854 ± 8 |
| KJ 5 | -- | | 731 ± 5 |

Dabei wurde die Konzentration der freien Maillard-Reaktionsprodukte CML: N^{ε}-Carboxymethyllysin (**2**); Pyr: Pyrralin (**3**) und MG-H: Methylhydroimidazolinon (**4**) untersucht. Dabei wurden sechs "biologisch" erzeugte Joghurtproben (BJ1-BJ6) mit fünf "konventionell" erzeugten Joghurtproben (KJ1-KJ5) verglichen. Wiederum konnte eine signifikante Abweichung in der Konzentration von Pyrralin (**3**) zwischen den "biologisch" erzeugten und "konventionell" erzeugten Joghurtproben nachgewiesen werden.

In einem weiteren Ausführungsbeispiel wurden stichprobenhaft Vergleichsuntersuchungen zwischen "konventionell" und "biologisch" erzeugter Buttermilch untersucht. Die Ergebnisse dieser Vergleichsuntersuchungen sind in der Tab. 6 dargestellt.

**Tab. 6**

| Probe | CML [pmol/ml] | MG-H [pmol/ml] | PYR [pmol/ml] |
|---|---|---|---|
| BBm 1 | 261 (1) | 152 ± 6 | 82 ± 6 |
| BBm 2 | - | 185 ± 39 | 242 ± 32 |
| BBm 3 | 265 ± 20,6 | - | - |
| BBm 4 | - | 152 ± 17 | 483 ± 55 |
| KBm 1 | 74 ± 64,4 | 214 ± 4 | 924 ± 130 |
| KBm 2 | 315 ± 419,4 | 205 ± 6 | 477 ± 48 |
| KBm 3 | - | 150 ± 14 | 990 ± 143 |

Dabei wurden vier "biologisch" erzeugte Buttermilchproben (BBm1-BBM4) mit drei "konventionell" erzeugten Buttermilchproben (KBm1-KBM4) verglichen. Wiederum konnte eine signifikante Abweichung in der Konzentration von Pyrralin (**3**) zwischen den "biologisch" erzeugten und "konventionell" erzeugten Buttermilchproben nachgewiesen werden.

In einem weiteren Ausführungsbeispiel wurden stichprobenhaft Vergleichsuntersuchungen zwischen "konventionell" und "biologisch" erzeugtem Quark untersucht. Die Ergebnisse dieser Vergleichsuntersuchungen sind in der Tab. 7 dargestellt.

**Tab. 7**

| Probe | CML (2) [pmol/ml] | MG-H (4) [pmol/ml] | PYR (3) [pmol/ml] |
|---|---|---|---|
| BQ1 | - | 66 ± 15 | 57 ± 4 |
| BQ 2 | - | 98 ± 3 | 34 ± 3 |
| BQ3 | - | - | 74 ± 3 |
| BQ4 | 320 ± 46 | - | - |
| KQ 1 | - | - | 665 ± 103 |
| KQ 2 | - | 87 ± 9 | 474 ± 4 |
| KQ3 | - | 202 ± 23 | 1057 ± 19 |
| KQ4 | 295 ± 45 | - | - |

Dabei wurden vier "biologisch" erzeugte Quarkproben (BQ1-BQ4) mit vier "konventionell" erzeugten Quarkproben (KQ1-KQ4) verglichen. Wiederum konnte eine signifikante Abweichung in der Konzentration von Pyrralin (**3**) zwischen den "biologisch" erzeugten und "konventionell" erzeugten Quarkproben nachgewiesen werden.

In einem weiteren Ausführungsbeispiel wurden stichprobenhaft Vergleichsuntersuchungen zwischen "konventionell" und "biologisch" erzeugtem Frischkäse untersucht. Die Ergebnisse dieser Vergleichsuntersuchungen sind in der Tab. 8 dargestellt.

**Tab. 8:**

| Probe | CML (2) [pmol/ml] | MG-H (4) [pmol/ml] | PYR (3) [pmol/ml] |
|---|---|---|---|
| BF 1 | 690 ± 131 | -- | 151 ± 2 |
| KF 1 | 655 ± 80 | 57 ± 6 | 466 ± 3 |

Dabei wurde in einem ersten Test eine "biologisch" erzeugte Frischkäseprobe (BF1) mit einer "konventionell" erzeugten Frischkäseprobe (KF1) verglichen. Wiederum konnte eine erhebliche Abweichung in der Konzentration von Pyrralin (3) zwischen den "biologisch" erzeugten und "konventionell" erzeugten Frischkäseprobe nachgewiesen werden. Diese Vergleichsuntersuchung geben einen deutlichen Hinwies darauf, dass auch für Frischkäseproben Pyrralin (3) als Markersubstanz für die Bewertung "biologisch" und "konventionell" erzeugter Frischkäse geeignet erscheint.

In einem weiteren Ausführungsbeispiel wurden stichprobenhaft Vergleichsuntersuchungen zwischen "konventionell" und "biologisch" erzeugtem Käse untersucht. Die Ergebnisse dieser Vergleichsuntersuchungen sind in der Tab. 9 dargestellt.

**Tab. 9**

| Probe | CML (2) [pmol/ml] | MG-H (4) [pmol/ml] | PYR (3) [pmol/ml] |
|---|---|---|---|
| BKä 1 | - | - | 118 ± 2,9 |
| KKä 1 | - | 26 ± 1,0 | 487 ± 17,7 |
| KKä 2 | - | - | 382 ± 12,0 |

Dabei wurde in einem ersten Test eine "biologisch" erzeugte Käseprobe (BKä1) mit zwei "konventionell" erzeugten Käseprobe (KKä1 und KKä2) verglichen. Wiederum konnte eine erhebliche Abweichung in der Konzentration von Pyrralin (**3**) zwischen den "biologisch" erzeugten und "konventionell" erzeugten Käseprobe nachgewiesen werden. Aufgrund der geringen Datenmenge ist hierbei noch keine abschließende statistische Bewertung möglich, jedoch gibt diese Vergleichsuntersuchung einen deutlichen Hinwies darauf, dass auch für Käseproben Pyrralin (**3**) als Markersubstanz für die Bewertung "biologisch" und "konventionell" erzeugter Käsesorten geeignet erscheint.

### Zitierte Nichtpatentliteratur

Ahmed, N., Mirshekar-Syahkal, B., Kennish, L., Karachalias, N., Babaei-Jadidi, R., Thornalley P. J. Assay of advanced glycation endproducts in selected beverages and food by liquid chromatography with tandem mass spectrometric detection. Molecular nutrition & food research 2005, 49, 691-699.
Dean, W., Dixon, W., Simplified statistics for small numbers of observations. Anal. Chem. 1951, 23, 636-638.
FIBL (2014), Fütterungsrichtlinie 2014 nach Bio Suisse, Forschungsinstitut für den biologischen Landbau (FIBL), online unter https://www.fibl.org/de/shop/artikel/c/richtlinien/p/1398-fuetterung.html, abgerufen am 21.07.2014
Förster, A., Henle, T., Glycation in food and metabolic transit of dietary studies on urinary excretion of pyrraline. Biochem. Soc. Trans. 2003, 31, 1383-1385
Förster, A., Kühne, Y., Henle, T., Studies on absorption and elimination of dietary Maillard reaction products. Annals N. Y. Acad. Sci. 2005, 1043, 474-481
Hellwig, M., Geißler, S., Peto, A., Knütter, I., Brandsch, M., Henle, T., Transport of free and peptide-bound pyrraline at intestinal and renal epithelial cells. J. Agric. Food Chem. 2009, 57, 6474-6480
Hellwig, M., Geissler, S., Matthes, R., Peto, A., Silow, C., Brandsch, M., Henle, T., Synthesis and intestinal transport of free and peptide-bound amino acid derivatives resulting from glycation reactions. ChemBioChem 2011, 12, 1270-1279
Hellwig, M., Matthes, R., Peto, A., Löbner, J., Henle, T., N-e-Fructosyllysine and N-e-carboxymethyllysine, but not lysinoalanine, are available for absorption after simulated gastrointestinal digestion. Amino Acids 2014, 46, 289-299
Henle, T., Protein-bound advanced glycation endproducts (AGEs) as bioactive amino acid derivatives in foods. Amino Acids 2005, 29, 313-322
Milchindustrieververband 03.04.2014, online unter http://www.milchindustrie.de/marktdaten/erzeugung/milchanlieferung-biomilch/abgerufen am 21.07.2014
Molkentin, J., Applicability of organic milk indicators to the authentication of processed products. Food Chem. 2013, 137, 13725-13730.
Molkentin, J., Giesemann, A., Differentiation of organically and conventionally produced milk by stable isotope and fatty acid analysis. Anal. Bioanal. Chem. 2007, 388,297-305
Poulsen, M.W., Hedegaard, R.V., Andersen, J.M., de Courten, B., Bügel, S., Nielsen, J., Skibsted, L.H., Dragsted, L.O., Advanced glycation endproducts in food and their effects on health. Food Chem. Toxicol. 2013, 60, 10-37.
Thornally, P. J., Battah, S., Ahmed, N., Karachalias, N., Agalou, S., Babaei-Jadidi, R., Dawnay A. Quantitative screening of advanced glycation endproducts in cellular and extracellular proteins by tandem mass spectrometry. Biochem J. 2003, 375, 581-592.
Tukey, J.W. (1977) Exploratory Data Analysis, Reading, Mass, Addison-Wesley

## Patentansprüche

1. Verfahren zur Bestimmung von gemäß Verordnung (EG) Nr. 834/2007 erzeugten Molkereiprodukten umfassend die Schritte:
- Analyse der zu bewertenden Molkereiprodukt-Probe,
- Identifizierung von zumindest einem Glykierungsprodukt in der zu bewertenden Molkereiprodukt-Probe,
- Quantifizierung des identifizierten Glykierungsprodukts mit zumindest einer Referenzsubstanz,
- Ermittlung signifikanter Abweichungen zwischen quantifiziertem Glykierungsprodukt und produktspezifischen Referenzwerten,
wobei als signifikante Abweichung des Gehalts an Glykierungsprodukt in der Molkereiproduktprobe eine Abweichung um das aus der Standardabweichung gerechnete Vertrauensintervall des Referenzwertes anzusehen ist, und wobei das zu identifizierende Glykierungsprodukt Pyrralin (3) und/oder Methylglyoxal Hydroimidazolon (4) ist.

2. Verfahren nach Anspruch 1, **dadurch gekennzeichnet, dass** zumindest zwei Glykierungsprodukte in der zu bewertenden Proben identifiziert werden.

3. Verfahren nach einem der vorhergehenden Ansprüche, **dadurch gekennzeichnet, dass** die Identifizierung des zumindest einen Glykierungsprodukts mittels Massenspektrometrie erfolgt.

4. Verwendung eines Verfahrens nach einem der Ansprüche 1 bis 3 zur Unterscheidung von "biologisch" und nicht gemäß Verordnung (EG) Nr. 834/2007 erzeugter, "konventioneller" Milch und daraus hergestellter Molkereiprodukte.

## Claims

1. Method for determination of dairy products produced in accordance with Regulation (EC) No. 834/2007 comprising the steps:
- Analysis of the dairy product sample to be evaluated,
- Identification of at least one glycation product in the dairy product sample to be evaluated,
- Quantification of the identified glycation product with at least one reference substance,
- Determination of significant deviations between quantified glycation product and product-specific reference values,
wherein a significant deviation of the glycation product content in the dairy product sample is a deviation by the confidence interval calculated from the standard deviation of the reference value, and
wherein the identified glycation product is pyrraline (3) and/or methylglyoxal hydroimidazolone (4).

2. The method according to claim 1, **characterized in that** at least two glycation products are identified in the sample to be evaluated.

3. Method according to one of the preceding claims, **characterized in that** the identification of the at least one glycation product is done by mass spectrometry.

4. Use of a method according to any of the claims 1 to 3 for distinction between "organic" and not in accordance with Regulation (EC) No 834/2007 produced, "conventional" milk and dairy products made of the same.

## Revendications

1. Procédé de détermination des produits laitiers produits conformément au règlement (CE) n° 834/2007, comprenant les étapes:
- à analyser l'échantillon de produit laitier à évaluer,
- à identifier au moins un produit de glycation dans l'échantillon de produit laitier à évaluer,
- à quantifier le produit de glycation identifié avec au moins une substance de référence,
- à déterminer des écarts significatifs entre le produit de glycation quantifié et les valeurs de référence spécifiques au produit,
dans lequel un écart par rapport à l'intervalle de confiance de la valeur de référence calculé à partir de l'écart type doit être considéré comme un écart significatif de la teneur en produit de glycation dans l'échantillon de produit laitier, et dans lequel le produit de glycation à identifier est la pyrraline (3) et/ou la méthylglyoxal hydroimidazolone (4).

2. Procédé selon la revendication 1, **caractérisé en ce qu'**au moins deux produits de glycation sont identifiés dans l'échantillon à évaluer.

3. Procédé selon l'une quelconque des revendications précédentes, **caractérisé en ce que** l'identification de l'au moins un produit de glycation est réalisée par spectrométrie de masse.

4. Utilisation d'un procédé selon l'une quelconque des revendications 1 à 3 pour faire la distinction entre un lait « biologique » et un lait « conventionnel » produit non conformément au règlement (CE) n° 834/2007 et les produits laitiers dérivés de celui-ci.
